# EUROPEAN PATENT APPLICATION

(11) **EP 2 034 025 A1**
(43) Date of publication of application: **11.03.2009**
(21) Application number: 07115647.5
(22) Date of filing: 04.09.2007
(51) Int. Cl.: C12N 15/85, A61K 48/00

(54) **Inflammation-induced anti-inflammatory gene expression**

(71) Applicant: Freie Universität Berlin, 14195 Berlin (DE)
(72) Inventor: Schmidt, Michael F.G. Prof. Dr., 14554 Seddiner See (DE); Rachakonda, Sivaramakrishna P. Dr., 12351 Berlin (DE)
(74) Representative: Baumgärtel, Gunnar

(57) **Abstract**

According to one embodiment of the present invention, a self regulating DNA expression vector is provided that allows for the inflammation-specific expression of immunologically or metabolically active polypeptide gene products.
The gene expression construct comprises a RNA polymerase promoter sequence that is induced by inflammation and which is operable in a mammalian cell, a transcribed sequence under the control of said promoter sequence, encoding a transcription product that has the ability to modulate the immune response, and/or encoding the mRNA for a polypeptide gene product that has the ability to modulate the immune response.

SEQUENCE LISTING

<110> Freie Universität Berlin

<120> Inflammation-induced anti-inflammatory gene expression

<130> FU 114

<160> 2

<170> PatentIn version 3.3

<210> 1
<211> 1240
<212> DNA
<213> Canis familiaris

<400> 1
tcctctctct ctctctctct catttcgccc ttacatcacc aaccaaaaga tattaaataa 60

aagcaaagga cgatgaaatt ttaaattgtc aaaatcatcc gtcttttcca cccattctac 120

tcacttcctc atcaaagtat gttccaaaat gagaatagaa aatttgcact cttaagccca 180

attgaaaaaa gtaaatgcaa tgttgtattt tgatccaccg tcacagtcct cctcgtctct 240

gaaaagtggg cagaaagaca aaaaaagcaa gcttaaaaaa aaaaaaaact agtttcttct 300

ccgtgtatct cctgtgaagg gctggtggcc aaagtggtga aagaaatgca cgaaagcaga 360

tgtgctgagc ttttagcatc ccggcagatc cctcatcaca agaggggaaa cacggtagct 420

caagctcgga ccgactgttg gcaggatcgc cgcttaggag cagtcctggg gtgaggaacc 480

aacagcttac agatgtgcac actgactcca gctattctat ttcattcagc caaaattttt 540

agaaaatcgt aatgtatgga tgtatgtgct acacgtacac taggtgacgg gtcgccacga 600

acatcatgac atgtttgaca tacatgacgt aacccgtgtc agcctttcct ggccccgtct 660

gtccagactg tgactcctcg ggcccgcaaa gacctgggca tgagacgcgg gggtggccgc 720

gcggggagat tccctgcgtc ctgggacctc cggccgctcg gattccggga gaggaggccc 780

cggattctcc ctcccggcag ggggacccgc cgcggcgccc gcgcgctctc cgaagctccc 840

ggtcaaagac tgcggagaaa gggatccggc gccaatcgcc ctccggaacc gaggggggca 900

attcgctttg cacccccttg gggaagtccg aggcagcttc tcgggtttcc gattttctca 960

tttgcgtgga gtcaaaaaaa aaaaaaaaat ccttacgcaa ttattaagta gacaggggga 1020

ggggagagtg atggggggga ggggggtgga aagaaacagt catgcgtcac gtgggcttag 1080

ttatcatgca taaaaggaag gttccgtccg ttagcgacta gttgtcaaac aacttgcagc 1140

gagcgtctgg agcacgctcg ggaactccgc acagcgcctg cctcctccag cgccgcagcc 1200

ccgagcccag gacgggaacg cctccgccgc cgcctccgcc 1240

<210> 2
<211> 5001
<212> DNA
<213> artificial

<220>
<223> self regulated vector with minimal length COX-2 promoter
construct, expressing the canine IL-4 (cIL-4) encoding sequence.

<400> 2
tagttattaa tcctctctct ctctctctct ctcatttcgc ccttacatca ccaaccaaaa 60
gatattaaat aaaagcaaag gacgatgaaa ttttaaattg tcaaaatcat ccgtcttttc 120
cacccattct actcacttcc tcatcaaagt atgttccaaa atgagaatag aaaatttgca 180
ctcttaagcc caattgaaaa aagtaaatgc aatgttgtat tttgatccac cgtcacagtc 240
ctcctcgtct ctgaaaagtg ggcagaaaga caaaaaaagc aaacttaaaa aaaaaaaaaa 300
aactagtttc ttctccgtgt atctcctgtg aagggctggt ggccaaagtg gtgaaagaaa 360
tgcacgaaag cagatgtgct gagcttttag catcccggca gatccctcat cacaagaggg 420
gaaacacggt agctcaagct cggaccgact gttggcagga tcgccgctta ggagcagtcc 480
tggggcgagg aaccaacagc ttacagatgt gcacactgac tccagctatt ctatttcatt 540
cagccaaaat ttttagaaaa tcgtaatgta tggatgtatg tgctacacgt acactaggtg 600
acgggtcgcc acgaacatca tgacatgttt gacatacatg acgtaacccg tgtcagcctt 660
tcctggcccc gtctgtccag actgtgactc ctcgggcccg caaagacctg ggcatgagac 720
gcgggggtgg ccgcgcgggg agattccctg cgtcctggga cctccggccg ctcggattcc 780
gggagaggag gccccggatt ctccctcccg gcagggggac ccgccgcggc gcccgcgcgc 840
tctccgaagc tcccggtcaa agactgcgga gaaagggaat ccggcgccaa tcgccctccg 900
gaaccgaggg gggcaattcg ctttgcaccc ccttggggaa gtccgaggca gcttctcggg 960
tttccgattt tctcatttgc gtggagtcaa aaaaaaaaaa aaaaatcctt acgcaattat 1020
taagtagaca gggggagggg agagtgatgg gggggagggg ggtggaaaga aacagtcatg 1080
cgtcacgtgg gcttagttat catgcataaa aggaaggttc cgtccgttag cgactagttg 1140
tcaaacaact tgcagcgagc gtctggagca cgctcgggaa ctccgcacag cgcctgcctc 1200
ctccagcgcc gcagccccga gcccaggacg ggaacgcctc cgccgccgcc tccgccggat 1260
ccgccaccat gggtctcacc tcccaactga ttccaactct ggtctgctta ctagcactca 1320
ccagcacctt tgtccacgga cataacttca atattactat taaagagatc atcaaaatgt 1380
tgaacatcct cacagcgaga aacgactcgt gcatggagct gactgtcaag gacgtcttca 1440
ctgctccaaa gaacacaagc gataaggaaa tcttctgcag agctgctact gtactgcggc 1500
agatctatac acacaactgc tccaacagat atctcagagg actctacagg aacctcagca 1560
gcatggcaaa caagacctgt tctatgaatg aaatcaagaa gagtacactg aaagacttct 1620
tggaaaggct aaaagtgatc atgcagaaga aatactacag gcattgagcg gccgcgactc 1680
tagatcataa tcagccatac cacatttgta gaggttttac ttgctttaaa aaacctccca 1740
cacctccccc tgaacctgaa acataaaatg aatgcaattg ttgttgttaa cttgtttatt 1800
gcagcttata atggttacaa ataaagcaat agcatcacaa atttcacaaa taaagcattt 1860
ttttcactgc attctagttg tggtttgtcc aaactcatca atgtatctta aggcgtaaat 1920
tgtaagcgtt aatattttgt taaaattcgc gttaaatttt tgttaaatca gctcattttt 1980

taaccaatag gccgaaatcg gcaaaatccc ttataaatca aaagaataga ccgagatagg 2040
gttgagtgtt gttccagttt ggaacaagag tccactatta aagaacgtgg actccaacgt 2100
caaagggcga aaaaccgtct atcagggcga tggcccacta cgtgaaccat caccctaatc 2160
aagttttttg gggtcgaggt gccgtaaagc actaaatcgg aaccctaaag ggagcccccg 2220
atttagagct tgacggggaa agccggcgaa cgtggcgaga aaggaaggga agaaagcgaa 2280
aggagcgggc gctagggcgc tggcaagtgt agcggtcacg ctgcgcgtaa ccaccacacc 2340
cgccgcgctt aatgcgccgc tacagggcgc gtcaggtggc acttttcggg gaaatgtgcg 2400
cggaacccct atttgtttat ttttctaaat acattcaaat atgtatccgc tcatgagaca 2460
ataaccctga taaatgcttc aataatattg aaaaaggaag agtcctgagg cggaaagaac 2520
cagctgtgga atgtgtgtca gttagggtgt ggaaagtccc caggctcccc agcaggcaga 2580
agtatgcaaa gcatgcatct caattagtca gcaaccaggt gtggaaagtc cccaggctcc 2640
ccagcaggca gaagtatgca aagcatgcat ctcaattagt cagcaaccat agtcccgccc 2700
ctaactccgc ccatcccgcc cctaactccg cccagttccg cccattctcc gccccatggc 2760
tgactaattt tttttattta tgcagaggcc gaggccgcct cggcctctga gctattccag 2820
aagtagtgag gaggcttttt tggaggccta ggcttttgca aagatcgatc aagagacagg 2880
atgaggatcg tttcgcatga ttgaacaaga tggattgcac gcaggttctc cggccgcttg 2940
ggtggagagg ctattcggct atgactgggc acaacagaca atcggctgct ctgatgccgc 3000
cgtgttccgg ctgtcagcgc aggggcgccc ggttcttttt gtcaagaccg acctgtccgg 3060
tgccctgaat gaactgcaag acgaggcagc gcggctatcg tggctggcca cgacgggcgt 3120
tccttgcgca gctgtgctcg acgttgtcac tgaagcggga agggactggc tgctattggg 3180
cgaagtgccg gggcaggatc tcctgtcatc tcaccttgct cctgccgaga aagtatccat 3240
catggctgat gcaatgcggc ggctgcatac gcttgatccg gctacctgcc cattcgacca 3300
ccaagcgaaa catcgcatcg agcgagcacg tactcggatg gaagccggtc ttgtcgatca 3360
ggatgatctg gacgaagagc atcaggggct cgcgccagcc gaactgttcg ccaggctcaa 3420
ggcgagcatg cccgacggcg aggatctcgt cgtgacccat ggcgatgcct gcttgccgaa 3480
tatcatggtg gaaaatggcc gcttttctgg attcatcgac tgtggccggc tgggtgtggc 3540
ggaccgctat caggacatag cgttggctac ccgtgatatt gctgaagagc ttggcggcga 3600
atgggctgac cgcttcctcg tgctttacgg tatcgccgct cccgattcgc agcgcatcgc 3660
cttctatcgc cttcttgacg agttcttctg agcgggactc tggggttcga aatgaccgac 3720
caagcgacgc ccaacctgcc atcacgagat ttcgattcca ccgccgcctt ctatgaaagg 3780
ttgggcttcg gaatcgtttt ccgggacgcc ggctggatga tcctccagcg cggggatctc 3840
atgctggagt tcttcgccca ccctaggggg aggctaactg aaacacggaa ggagacaata 3900
ccggaaggaa cccgcgctat gacggcaata aaaagacaga ataaaacgca cggtgttggg 3960
tcgtttgttc ataaacgcgg ggttcggtcc cagggctggc actctgtcga taccccaccg 4020
agaccccatt ggggccaata cgcccgcgtt tcttcctttt ccccacccca ccccccaagt 4080

tcgggtgaag gcccagggct cgcagccaac gtcggggcgg caggccctgc catagcctca 4140
ggttactcat atatacttta gattgattta aaacttcatt tttaatttaa aaggatctag 4200
gtgaagatcc tttttgataa tctcatgacc aaaatccctt aacgtgagtt ttcgttccac 4260
tgagcgtcag accccgtaga aaagatcaaa ggatcttctt gagatccttt ttttctgcgc 4320
gtaatctgct gcttgcaaac aaaaaaacca ccgctaccag cggtggtttg tttgccggat 4380
caagagctac caactctttt tccgaaggta actggcttca gcagagcgca gataccaaat 4440
actgtccttc tagtgtagcc gtagttaggc caccacttca agaactctgt agcaccgcct 4500
acatacctcg ctctgctaat cctgttacca gtggctgctg ccagtggcga taagtcgtgt 4560
cttaccgggt tggactcaag acgatagtta ccggataagg cgcagcggtc gggctgaacg 4620
gggggttcgt gcacacagcc cagcttggag cgaacgacct acaccgaact gagataccta 4680
cagcgtgagc tatgagaaag cgccacgctt cccgaaggga gaaaggcgga caggtatccg 4740
gtaagcggca gggtcggaac aggagagcgc acgagggagc ttccaggggg aaacgcctgg 4800
tatctttata gtcctgtcgg gtttcgccac ctctgacttg agcgtcgatt tttgtgatgc 4860
tcgtcagggg ggcggagcct atggaaaaac gccagcaacg cggccttttt acggttcctg 4920
gccttttgct ggccttttgc tcacatgttc tttcctgcgt tatcccctga ttctgtggat 4980
aaccgtatta ccgccatgca t 5001

## Description

Rheumatoid arthritis (RA) and osteoarthritis (OA) are the common forms of arthritis. The pathology involves joint swelling and synovial inflammation. The reasons for the initiation of these disease processes is not yet clear, but the mechanism of inflammation that follows the disease is well understood.

It has been established that Th1 cells represented by their capacity to synthesize interleukin IL-1β and tumour necrosis factor TNFα trigger the inflammatory cascade by binding to their respective receptors. Pain is the common symptom resulting from the inflammation cascade, which is similar in both forms of the disease.

The widely followed symptomatic treatment of RA involves pain killers, prominently non-steroidal anti-inflammatory drugs (NSAIDs). Compounds are available that are supposed to inhibit the cyclooxygenase-2 (COX-2) enzyme more or less selectively, which is a key enzyme in the synthesis of PGE2 which causes pain. Treatment with NSAIDs or selective COX-2 inhibitors has proven to be successful in controlling pain, however has restricted use due to these compounds' side effects upon long term use.

NSAIDs, however, also block the synthesis of cyclooxygenase-1 (COX-1), which is constitutively expressed and has role in homeostasis. In contrast, COX-2 is an inducible enzyme and is known to be induced in inflammatory conditions. Some reports show that COX-2 also has a significant role in developing prostaglandin D2 (PGD2), an isoform of prostaglandin PGE2, which is involved in anti-inflammatory conditions. This shows that complete inhibition of COX-2 may deprive the system of its natural course of anti-inflammatory mechanisms.

Since inflammatory pathways of RA/OA have shown a clear imbalance between Th1 and Th2 cell populations, production of Th2 cells could restore the homeostasis in the knee joint. It has been shown that interleukins IL-4, IL-10 or IL-13 can counter the inflammation triggered by IL-1β and TNFα, and hence are potential candidates for gene therapy in RA/OA. Further expression of IL-1Ra or treatment with TNFα monoclonal antibodies also blocked the inflammation cascade. Although candidate genes for interference with the inflammatory process have been identified, approaches of the kind commonly labelled as "gene therapy" fall short of meeting the requirement of a mechanism of expression of the therapeutic gene that is amenable to regulation. Typically, a therapeutic gene should be inducible only in the presence of inflammation and should be moreover self-regulating i.e., initiating a negative feed back mechanism.

One objective of the current invention is to provide means and methods to selectively inhibit the inflammatory mechanism of COX-2 in tissue undergoing an inflammatory reaction, rather than to inhibit COX-2 completely.

Another objective of the current invention is to provide means and methods to suppress or attenuate the inflammatory reaction in tissue by expressing genes encoding anti-inflammatory gene products in a self-regulated fashion.

These objectives are attained by the methods and nucleic acid sequences as specified in the independent claims.

### Definitions:

Within the context of the current specification, the word "inflammation" shall mean a local increase in lymphocytes such as monocytes, T-cells, B-cells, leukocytes, natural-killer (NK) cells, macrophages or the like; a local increase of interleukins, interferons, tumor necrosis factor or prostaglandins, leukotrienes or other small signalling molecule concentration.

Within the context of the current specification, the words "a RNA polymerase promoter sequence that is induced by inflammation and which is operable in a mammalian cell" shall mean a DNA sequence that, when present in a mammalian cell of an inflamed tissue, can drive -provided eventually necessary other enhancing or modulating factors- the transcription of a transcribed sequence under its control by RNA polymerase at a level significantly above the transcription level which would be observed without the presence of the inflamed tissue or the Rel/nuclear factor-kb (NF-kb) transcription factor associated with inflammation. If the transcription product of the transcribed sequence is an mRNA, the RNA polymerase is likely to be RNA polymerase II.

According to the invention, a promoter sequence activated in tissue undergoing inflammation, or in cells reacting to inflammation, is employed to regulate the expression level of a gene encoding an anti-inflammatory gene product.

According to one embodiment of the invention, such promoter sequence activated in tissue undergoing inflammation is a COX-2-specific promoter sequence derived from a COX-2 genomic DNA. An anti-inflammatory gene product can be interleukin 4 (IL-4), interleukin 10 or 13, a soluble IL-1Ra molecule, a TNFα-monoclonal antibody or other immune-modulatory or metabolically active polypeptides known in the art.

According to one embodiment of the present invention, a self regulating DNA expression vector is provided that allows for the inflammation-specific expression of immunologically or metabolically active polypeptide gene products.

According to one aspect of the invention, the induction of gene expression of an anti-inflammatory gene product such as IL-4 can trigger a negative feed back mechanism that attenuates the inflammation and thereby decreases expression of genes regulated by the COX-2 promoter. According to this aspect of the invention, COX-2 promoter constructs allow to significantly down-regulate the expression of a plurality of inflammatory cytokines.

According to another aspect of the invention, genes encoding anti-inflammatory gene products such as IL-4 that are regulated by an inflammation sensitive COX-2 promoter will be predominantly expressed in tissue undergoing inflammation. This results in expression of such genes in a regulated fashion, rather than uncontrolled over-expression from virally-derived promoter sequences such as CMV, as has been the case in traditional approaches of gene therapy. Thereby, the likelihood of side effects is significantly reduced.

According to another aspect of the invention, a responsive and regulatory vector (SeqID 002) was constructed using a COX-2 promoter (SeqID 001).

According to another aspect of the invention, the so-called COX-2 promoter that regulates transcription of the cyclooxygenase 2 protein was used to demonstrate the utility of the general concept. COX enzymes mediate the synthesis of PGH2, a precursor of PGE2 that is responsible for the increased sensitivity perceived as pain during inflammation. Moreover, COX-2 is one of the downstream targets in the inflammation pathway. Furthermore, COX-2 has been shown to be up-regulated in IL-1β/ TNFα, Wnt-signalling and also Ras-signalling pathways, which are all functional in OA/RA, and other diseases such as type II diabetes. In fact over-expression of COX-2 is associated with majority of the chronic forms of inflammation that subsequently leads to cancer. A self regulated vector was designed using a minimal length COX-2 promoter construct and expressing the canine IL-4 (cIL-4) encoding sequence from it. IL-4 was chosen as an example due to its role in T and B cell differentiation and in initiating type II immune responses. Experimentally, inflammation was triggered in vitro in canine articular chondrocytes (CAC) using canine IL-1β and TNFα and the effect of canine IL-4 was studied on the inflammatory cascade. Our results show that COX-2 promoter is inducible and expresses IL-4 only in the presence of canine IL-1β and TNFα.

### Figures

- Fig. 1: shows the detection of cIL-4 expression in CAC by Western blot.
- Fig. 2: shows the detection of cIL-4 expression in CAC by Western blot.
- Fig. 3: shows the quantification of matrix metalloproteinases (MMPs) by quantitative real time PCR (qRT-PCR).
- Fig. 4: shows the quantification of interleukin-1 receptor antagonist (IL-1ra) and insulin like growth factor (IGF)-1 by qRT-PCR.
- Fig. 5:: shows the quantification of PGE2 synthase.
- Fig. 6:: shows a nitrite Assay. The (+) and (-) signs refer to samples with (+) or without (-) treatment with cytokines
- Fig. 7:: shows a PGE2 assay.

The invention is further illustrated by the following examples.

### Example 1:

### Construction of COX-2 promoter construct

To synthesize the canine COX-2 promoter, primers were designed according to the canine genome database. Genomic DNA was extracted from canine blood (boxer species) and a stretch of 2 kb specific to the COX-2 promoter region was amplified. This 2 kb amplicon was sequenced and found to be homologous to the sequence listed in the canine genome database. Of the 2 kb sequence, functionally active elements were found to be within the initial 900 bp (-900 to +1) by using genomatrix software. Taking this into consideration, a COX-2 promoter of 1.25 kb (-1147 to +93) length was chosen for the present study (Seq ID 001). For flexibility, an extra 250 bp length (-901 to -1147) and a part of the first exon region (+93 bp) of COX-2 gene was considered additional to the minimum length of promoter sequence of 900 bp.

The COX-2 promoter sequence thus provided was cloned into the plasmid vector pDsRed2-N1 (Clontech, USA) substituting the CMV promoter between the Ase I and Bam HI restriction sites. The DsRed2 is a red colored Reef Coral Fluorescent Protein obtained from *Discosoma* sp. DsRed is a member of a variety of Living Colors (Trademark of Clontech) Reef Coral Fluorescent Proteins that do not need any substrate for development of fluorescence and are ideal for live cell assays. Cloning of COX-2 promoter into pDsRed2-N1 resulted in the vector referred to as "pCOX-2-DsRed", which was used for performing reporter assay experiments. Subsequently, the DsRed2 gene sequence was substituted with the IL-4 gene coding sequence (Accession no: AF239917) to form pCOX-2-IL4 plasmid. This vector has the backbone of the pDsRed2-N1 vector from Clontech. The entire vector sequence is given in Seq ID 002.

For the reporter assay CAC (approx., 10⁶ cells) were grown in cell culture at 37°C with 5 % CO₂ using complete medium (DMEM with 10 % FCS and 1 % pencillin, streptomycin). These cells were transiently transfected with 8 µg of plasmid DNA of pCOX-2-DsRed construct. Transfection was done using Nucleofector technology from Amaxa. As a positive control, the intact pDsRed2-N1 plasmid DNA with CMV promoter was transfected into CAC. The CAC with pCOX-2-DsRed were treated with or without 100 ng of IL-1β and 50 ng of TNFα in cell culture medium for a period of 48 -72 hours. After trypsinization the cells were FACS analysed using an Argon laser at 488 nm. Incidentally, fluorescence of DsRed2 protein was observed in CAC transfected with pCOX-2-DsRed and stimulated with IL-1β and TNFα. In contrast, no fluorescence could be observed in un-stimulated cells. However, CAC transfected with pDsRed2-N1 (positive control) showed fluorescence.

### Expression of IL-4 under control of the canine COX-2 promoter

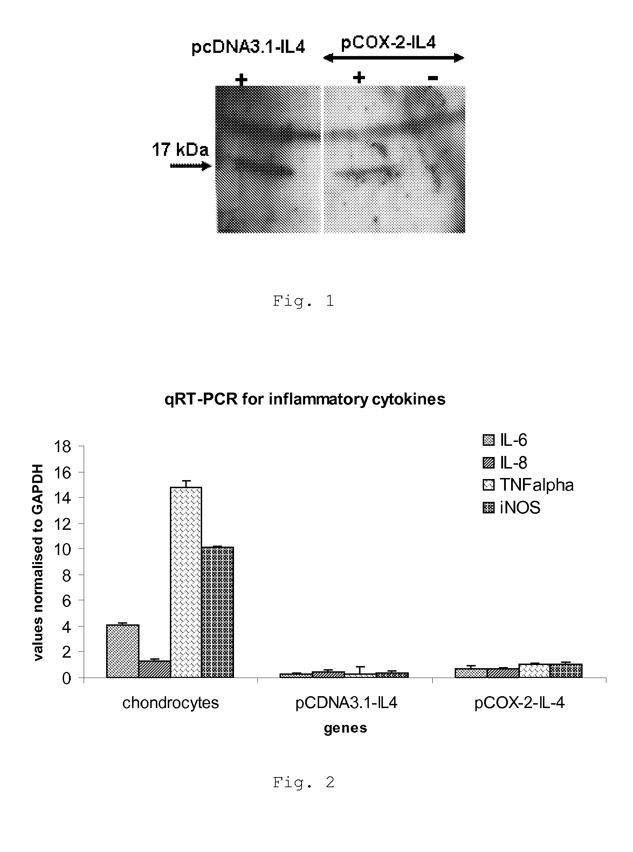

CAC (approx., 10⁶ cells) were transiently transfected with 8 µg of pCOX-2-IL4 constructs using either FuGENE 6 or Amaxa nucleofector and cells were then stimulated with cIL-1β and cTNFα as mentioned above. As a control, non-stimulated CAC that were either transfected with the above constructs independently or non-transfected cells were taken. After incubation with the cytokines for 72 hours, cells were lysed with RIPA buffer (1 % Triton X-100, 1 % deoxycholate, 0.1 % SDS, 0.15 M NaCl, 20 mM Tris, 10 mM EDTA, 10 mM iodoacetamide, 1 mM PMSF). The lysate was analyzed for IL-4 protein using canine IL-4 specific polyclonal antibody. Results of Western blot analysis clearly show the expression of IL-4 only in the transfected cells that were stimulated with cIL-1β and cTNFα (Fig. 1).

Fig. 1 shows the detection of cIL-4 expression in CAC by Western blot. CAC were transiently transfected with the pCOX-2-IL4 and treated with or without IL-1β and TNFα for 72 hours. The cell lysate was probed using cIL-4 specific polyclonal antibody. Expression of IL-4 (17 kDa) was detected only in the transfected cells that were stimulated with IL-1β and TNFα. (+) denotes CAC stimulated with 100 ng of IL-1β and 50 ng of TNFα and (-) denotes non-stimulated CAC.

### Example 2:

### pCOX-2-IL-4 downregulates inflammatory cytokines

IL-4 could be expressed from pCOX-2-IL4 construct only after stimulation by IL-1β and TNFα. At the same time, the expressed IL-4 is expected to negatively regulate the production of IL-1β and TNFα. To observe this regulation, CAC were transfected with either pCDNA3.1-IL4 or with pCOX-2-IL4. Non-transfected CAC served as a control. Control and transfected CAC were taken in duplicate. One set were treated with IL-1β and TNFα, while the other set left untreated. Subsequently, all cells were incubated at 37°C /5 % CO₂ for 72 hrs.

pCDNA3.1-IL4 has IL-4 cloned under the CMV promoter and this vector shows unregulated expression of IL-4 as expected.

The cell culture supernatant was taken for nitrite measurement by Griess reagent and the cell lysate was subjected to RNA extraction using TRIsure (Bioline GmbH, Germany). Gene quantification for various cytokines was done by qRT-PCR using Bio-Rad IQ5 real time PCR machine.

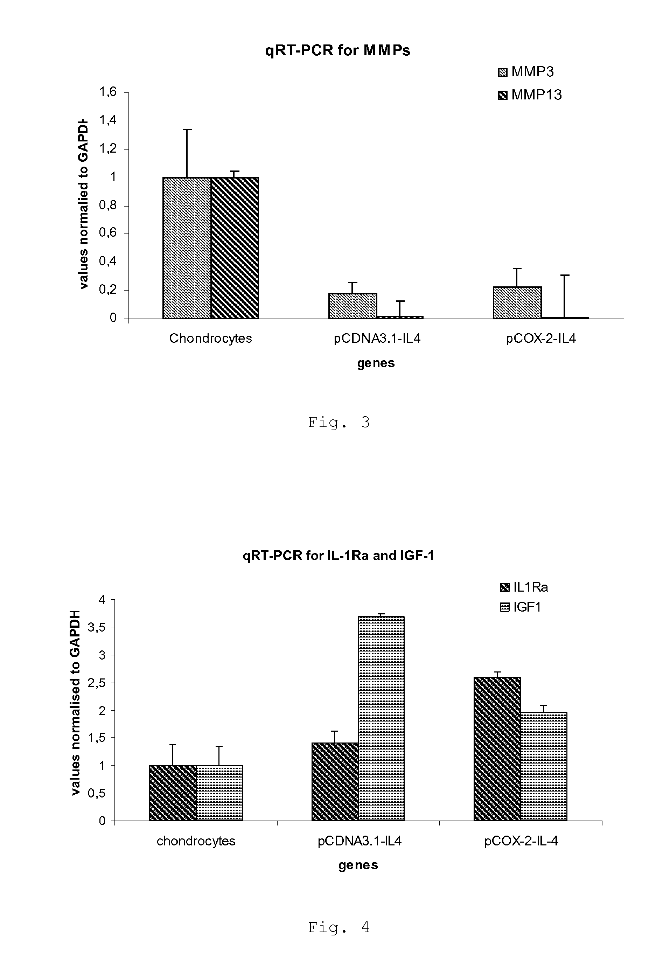

It was observed that pCOX-2-IL4 downregulates inflammatory mediators on comparable terms with that of pCDNA3.1-IL4. The down-regulation is evident when compared to CAC that were non-transfected but treated with IL-1β and TNFα (Fig. 2 and 3).

Fig. 2 shows the quantification of inflammatory cytokines by qRT-PCR.
Quantification of mRNA expression for TNFα, IL-6, IL-8 and iNOS was done relative to GAPDH expression. The effect of the pCOX-2-IL4 is compared with pCDNA3.1-IL4 and as well against the non-transfected CAC. Only the samples stimulated with 100 ng IL-1β and 50 ng of TNFα were taken for the study. A very low expression of inflammatory cytokines was observed in CAC showing IL-4 gene expression.

Fig. 3 shows the quantification of matrix metalloproteinases (MMPs) by qRT-PCR.
Quantification of mRNA expression for MMPs relative to GAPDH expression was done after stimulation with IL-1β and TNFα. The effect of the pCOX-2-IL4 is compared with pCDNA3.1-IL4 and as well against the untransfected CAC. Only the samples stimulated with 100 ng IL-1β and 50 ng of TNFα were taken for the study. Down-regulation of MMPs was evident because of expression of IL-4 in the respective samples.

### Example 3:

### Canine IL-4 up-regulates interleukin-1 receptor antagonist (IL-1Ra) and insulin like growth factor (IGF-1)

It has been found that cells transfected with IL-4 expressing constructs upon stimulation with IL-1β and TNFα for 72-96 hours produce IL-1Ra, and IGF-1. Up-regulation of IL-1Ra corresponds to down-regulation of IL-1β. Results are shown in Figure 4.

Fig. 4 shows the quantification of IL-1ra and IGF-1 by qRT-PCR. qRT-PCR demonstrates mRNA expression of IL-1ra and IGF-1 relative to GAPDH expression after stimulation with 100 ng IL-1β and 50 ng of TNFα using pCOX-2-IL4 construct. An increased expression of IL-1ra and IGF-1 was observed in both constructs.

### Example 4:

### IL-4 downregulates PGE2

COX-2 catalyses the conversion of arachidonic acid to PGH2, subsequently converted in a cell specific manner to various prostanoids like PGE2, PGD2, or PGF2 or PGI2. Of these forms, PGE2 is overexpressed during inflammation and is responsible for pain associated with arthritis. The conversion of PGH2 to PGE2 is carried out by PGE2 synthase which exists in two isoforms that are microsomal associated viz., mPGES-1 and -2 and one cytosolic form cPGES. Among these forms, it is mPGES-1 that is associated with inflammatory reactions. In the past, IL-4 has been shown to down-regulate mPGES-1 and thereby PGE2. Using pCOX-2-IL4 construct PGE2 should be down-regulated (to be verified) by quantifying mPGES-1.

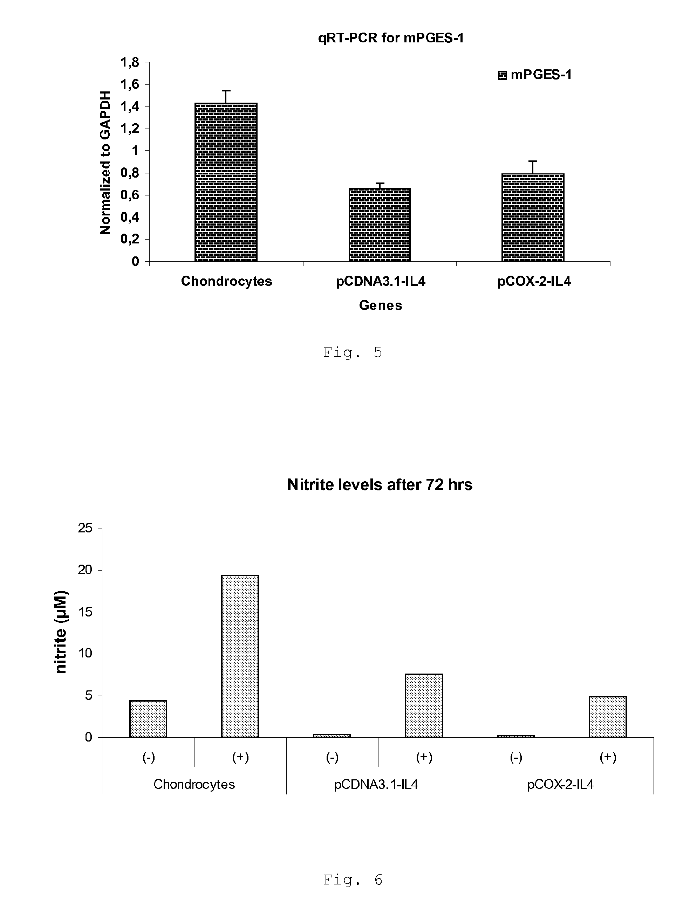

mRNA was extracted from CAC that had been transfected with pCDNA3.1-IL4 and pCOX-2-IL4, and stimulated with IL-1β and TNFα for 72 hours. As a control, non-transfected canine chondrocytes were used that were also stimulated with IL-1β and TNFα for 72 hours (Fig.5).

Fig. 5 shows the quantification of mPGES-1 by qRT-PCR.
mRNA expression was quantified for mPGES-1 relative to GAPDH expression after stimulation with 100 ng IL-1β and 50 ng of TNFα. A decreased expression of mPGES-1 was observed in gene constructs expressing IL-4 indicative of down-regulation of PGE2.

### Example 5:

### Inhibition of nitrite release by expression of IL-4

As mentioned previously, nitrite levels serve as good indicators of inflammation. Hence the cell culture supernatants from transfected and non-transfected control cells with or without stimulation were subjected to quantification of nitrite levels by the Griess reagent system. It was observed that nitrite levels are reduced in the presence of IL-4, which shows that iNOS is down-regulated.

This in turn would also downregulate the production of MMPs and would give a scope of cartilage regeneration (Fig. 6).

Fig. 6 shows the results of the Nitrite assay.
Cell culture supernatants from transfected and non transfected chondrocytes were assayed with or without stimulation with IL-1β and TNFα. The constructs that were transfected included pCDNA3.1-IL4 and pCOX-2-IL4. The control chondrocytes were not transfected with any plasmid DNA. The nitrite levels in supernatant were quantified using Griess reagent system and a reduction in nitrite levels were detected in those transfected with IL4 gene, but still stimulated. (+) denotes CAC stimulated with 100 ng IL-1β and 50 ng of TNFα and (-) denotes non-stimulated CAC.

Our results show that IL-4 expression in CAC leads to a down-regulation of the selected inflammatory cytokines. Moreover, expression of IL-4 under the COX-2 promoter occurs only in the presence of IL-1β and TNFα, which shows that pCOX-2-IL4, is responsive to inflammation.

### Example 6:

### PGE2 Assay

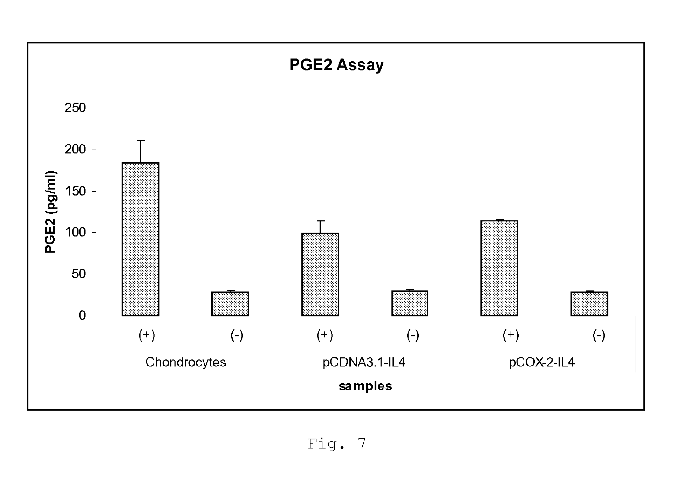

The cell culture supernatant was collected from the transfected and non-transfected control cells with or without stimulation was subjected to PGE2 quantification by Cayman's EIA kit using PGE2 monoclonal antibody. The results show that PGE2 is indeed down-regulated in the samples that were transfected with IL-4 constructs (pCDNA3.1-IL4 and pCOX-2-IL4). The result shown in Fig.7 confirms the data obtained from the NO assay and from quantification of mPGES-1 by qRT-PCR result (Fig.7).

Fig. 7 shows the result of the PGE2 assay.
The PGE2 levels in cell culture supernatants were quantified by Cayman's EIA kit according to manufacturer's instructions. A reduction in PGE2 levels were detected in those cells that were simulated and transfected with IL4 gene. (+) denotes CAC stimulated with 100 ng IL-1β and 50 ng of TNFα and (-) denotes non-stimulated CAC.

## Claims

1. Gene expression construct comprising
- a RNA polymerase promoter sequence that is induced by inflammation and which is operable in a mammalian cell,
- a transcribed sequence under the control of said promoter sequence, said transcribed sequence encoding a transcription product that has the ability to modulate the immune response, and/or encoding an mRNA for a polypeptide gene product that has the ability to modulate the immune response.

2. Gene expression construct according to claim 1, **characterized in that** the ability to modulate the immune system is an anti-inflammatory effect.

3. Gene expression construct according to claim 1 or 2, **characterized in that** the promoter sequence is a promoter sequence specific for COX-2.

4. Gene expression construct according to any of the preceding claims, **characterized in that** the promoter sequence comprises the sequence of Seq ID 001.

5. Gene expression construct according to any of the preceding claims, **characterized in that** the transcribed sequence encodes an interleukin, an interferon or a tumor necrosis factor or a soluble receptor to or a monoclonal antibody against an interleukin, an interferon or a tumor necrosis factor.

6. Gene expression construct according to any of the preceding claims, **characterized in that** the transcribed sequence encodes interleukin 4, interleukin 10, interleukin 13, a monoclonal antibody against tumor necrosis factor or IL-1Ra.

7. Gene expression construct according to any of the preceding claims, **characterized in that** the transcribed sequence encodes a protein active in dogs.

8. Gene expression construct according to any of the preceding claims, **characterized in that** the promoter is a promoter active in dogs.

9. A nucleic acid molecule comprising the sequence Seq ID 002.

10. Isolated mammalian cell comprising a gene expression construct or a nucleic acid molecule according to any of the above claims.

11. Use of a gene expression product or a nucleic acid molecule according to any of the above claims in the preparation of a pharmaceutical composition for the treatment of a disease.

12. Use according to claim 11, **characterized in that** the disease is an inflammatory disease.

13. Use according to claim 11 or 12, **characterized in that** the disease is at least one of rheumatoid arthritis, osteoarthritis, diabetes and cancer.

14. Use according to any of the preceding claims 11 to 13, **characterized in that** the disease is a disease in a mammal.

15. Use according to claim 14 **characterized in that** the mammal is a dog.

16. Use according to any of the preceding claims 11 to 13, **characterized in that** the disease is a disease in a human being.
